# EUROPEAN PATENT APPLICATION

(11) **EP 4 696 614 A1**
(43) Date of publication of application: **18.02.2026**
(21) Application number: 24857735.5
(22) Date of filing: 26.03.2024
(51) Int. Cl.: B65D 83/04, A61J 7/04

(54) **SMART MEDICINE BOX AND METHOD OF USING SAME**

(30) Priority: 31.08.2023 CN 202311110313
(71) Applicant: Kunshan Hi-Fortune Health Products Co., Ltd, Suzhou, Jiangsu 215000 (CN)
(72) Inventor: ZHI, Hongfa, Suzhou, Jiangsu 215000 (CN); FAN, Jianjiang, Suzhou, Jiangsu 215000 (CN)
(74) Representative: Murgitroyd & Company
(86) International application number: PCT/CN2024/083703
(87) International publication number: WO 2025/044183

(57) **Abstract**

A smart medicine box and a method of using same. The smart medicine box comprises: a medicine box, provided with a medicine outlet; a rotating frame, rotatably arranged in the medicine box, a plurality of sets of medicine cases distributed in the circumferential direction and capable of sliding in a reciprocating mode in the radial direction being arranged on the rotating frame, and the medicine cases being connected to the rotating frame by means of radially arranged springs; a driving module, arranged in the medicine box and composed of a rotating motor and a push rod motor, the rotating motor being used for driving the rotating frame to rotate such that a medicine case rotates to a medicine discharging position, and the push rod motor being used for driving a push rod to push the medicine case rotating to the medicine discharging position out of the medicine outlet; a medicine recognition module, fixedly arranged in the medicine box and used for recognizing medicine information; a face recognition module, fixedly arranged on the medicine box and used for recognizing user identity information; and a reminding module, fixedly arranged on the medicine box and used for reminding a user to take medicines or supplement medicines. The present solution can achieve multiple reminding functions such as medicine taking, dosage, medicine supplementing, and correct medicine taking, and is convenient for the elderly to use.

## Description

### TECHNICAL FIELD

The present disclosure relates to the field of artificial intelligence technologies, and particularly to an intelligent medicine box and a usage method thereof.

### BACKGROUND

With the rapid development of artificial intelligence (Al), the integration of AI and healthcare has become deeply rooted. Simultaneously, as Internet of Things technology continues to mature and be applied, combined with modern scientific technologies such as computer technology, internet communication technology, and automation technology, various intelligent medical products are continuously emerging. The market scale is constantly expanding, gradually changing people's production and daily lifestyles. As the pursuit of technological products continues to increase, people are no longer satisfied with the basic functions implemented by such products and often have higher requirements for the intelligence and user-friendliness of products.

With the rapid advancement of medical science, people are increasingly recognizing the importance of health. Especially in this fast-paced era, young adults may forget to take medicine due to busy work schedules, while the elderly may experience age-related memory decline, leading to forgotten doses or even overdosing because they cannot remember whether they have already taken their medication, or even taking the wrong medicine. A significant majority of patients are adversely affected by poor medication adherence-not following medical advice, or failing to take medicine on time and in the correct dosage. In this context, designing an intelligent medicine box that can cater to different users, precisely locate medication positions, and possess reminder functions is particularly important.

Many intelligent reminder medicine boxes on the market are based on internet technology, utilizing the coordination between mobile software and smart hardware to remind users to take medicine scientifically and on time. Essentially, these products are just traditional medicine boxes with added smart reminder functions. They still suffer from storage disorganization and do not solve problems such as the elderly taking the wrong medicine or missing doses. In terms of basic functionality, they are sometimes even inferior to simple reminder pillboxes. Furthermore, most products, in an effort to highlight their intelligence, become cumbersome and complex, making them unsuitable for the elderly population.

Therefore, addressing the shortcomings in the related art, it is necessary to design an intelligent medicine box and its usage method to resolve the aforementioned issues.

### SUMMARY OF THE DISCLOSURE

To overcome the shortcomings of the related art described above, the present disclosure aims to provide an intelligent medicine cabinet and its method of use.

To achieve the aforementioned objective and other related objectives, the technical solution provided by the present disclosure is as follows: An intelligent medicine box, including:
a medicine box body; wherein the medicine box body is arranged with a medicine storage compartment and a transition compartment that are in communication with each other, and the medicine box body is further arranged with a medicine outlet in communication with the transition compartment;
a rotatable frame, rotatably arranged in the medicine storage compartment; wherein the rotatable frame is arranged with a plurality of medicine cartridges that are distributed peripherally and capable of reciprocating sliding radially; the plurality of medicine cartridges and the rotatable frame are connected by springs that are radially arranged;
a drive module, fixedly arranged in the medicine box body and including a rotary motor and a push-rod electric motor; wherein the rotary motor is configured to drive the rotatable frame to rotate, causing one of the plurality of medicine cartridges to rotate to the medicine outlet; the push-rod electric motor is configured to drive a push rod to push the one of the plurality of medicine cartridges that has rotated to the medicine outlet out through the medicine outlet;
a medicine identification module, fixedly arranged in the transition compartment and configured to identify medicine information;
a facial recognition module, fixedly arranged on the medicine box body and configured to identify identity information of a user;
a prompt module, fixedly arranged on the medicine box body and configured to remind the user to take medicine or replenish medicine; and
a control module, fixedly arranged on the medicine box body and connected to the drive module, the medicine identification module, the facial recognition module, and the prompt module.

In some embodiments, the medicine box body is arranged with a medicine outlet door at the medicine outlet, and the medicine outlet door is installed via a torsion spring hinge.

In some embodiments, the rotatable frame includes a top plate, a bottom plate, and support plates; the top plate is configured as a circular plate structure, the bottom plate is configured as an annular plate structure, and the top plate and the bottom plate are arranged spaced apart vertically and facing each other and are fixedly supported by the support plates;
a bottom of the medicine box body is arranged with a plurality of ball seats distributed at equal angles peripherally; a bottom side of the bottom plate is arranged with an annular track correspondingly matching the plurality of ball seats.

In some embodiments, a top side of the bottom plate is arranged with a plurality of tracks distributed at equal angles peripherally; a bottom side of the top plate is arranged with a plurality of hanger wheels distributed at equal angles peripherally; the plurality of tracks and the plurality of hanger wheels are arranged in a one-to-one correspondence; a bottom side of each medicine cartridge is arranged with a pulley to match a corresponding track, and a top side of the medicine cartridge is arranged with a hanger rail to match a corresponding hanger wheel.

In some embodiments, an inner ring of the bottom plate is arranged with a first spring mounting base that is cylindrical; the first spring mounting base defines a plurality of first spring connection hook holes matching the plurality of tracks in a one-to-one correspondence;
the bottom side of the top plate is arranged with a second spring mounting base that is cylindrical; the second spring mounting base defines a plurality of second spring connection hook holes matching the plurality of hanger wheels in a one-to-one correspondence;
a first spring connection post is arranged in and on a bottom of each medicine cartridge, and the first spring connection post matches a corresponding first spring connection hook hole; a first spring is connected between the corresponding first spring connection hook hole and the first spring connection post;
a second spring connection post is arranged in and on a top of each medicine cartridge, and the second spring connection post matches a corresponding second spring connection hook hole; a second spring 14 is connected between the corresponding second spring connection hook hole and the second spring connection post.

In some embodiments, the drive module is fixedly arranged in the medicine box body via a bracket and disposed within an inner ring of the bottom plate, and the rotary motor is connected to a rotation center of the top plate in a transmission manner via a reduction gear.

In some embodiments, the push-rod electric motor is fixedly arranged in the medicine box body via the bracket and oriented toward the medicine outlet.

In some embodiments, medicine in one of the plurality of medicine cartridges is sheet-like or capsule-like medicine encapsulated in an aluminum-plastic blister pack; the medicine encapsulated in the aluminum-plastic blister pack is stored in the one of the plurality of medicine cartridges, with a bottom of the medicine supported on a bottom of the one of the plurality of medicine cartridges and a top of the medicine resting against a side wall of the one of the plurality of medicine cartridges; includtwo medicine identification modules are provided, which are respectively located on both sides of the one of the plurality of medicine cartridges.

In some embodiments, the medicine cartridge is made of transparent plastic, and a top side of the medicine box body further defines a medicine storage groove for storing medicine bottles.

A usage method based on the intelligent medicine box as above, including:
Operation 1: inputting the identity information of the user into a system through the facial recognition module; pushing one of the multiple medicine cartridges out of the medicine box body by the drive module, and placing medicine into the one of the multiple medicine cartridges; retracting the one of the multiple medicine cartridges into the medicine box body by the drive module; during a retraction process, automatically identifying the medicine information of the medicine in the one of the multiple medicine cartridges by the medicine identification module, and inputting the medicine information into the system; inputting a medication plan into the system through the control module;
Operation 2: according to the medication plan, reminding, by the system, the user to take medicine through the prompt module; in response to the user approaching the medicine box body, automatically identifying the identity information of the user by the facial recognition module; in response to the user being identified to be authorized, granting usage permission for the control module to the user; in response to the user being identified to be unauthorized, denying the usage permission for the control module to the user;
Operation 3: in response to medication use being authorized by the user through the control module, controlling, by the system, the rotary motor to drive the rotatable frame to rotate a corresponding medicine cartridge corresponding to a specified medicine to a medicine dispensing position based on the medicine information; controlling, by the system, the push-rod electric motor to drive the push rod to push the corresponding medicine cartridge from the medicine dispensing position into the transition compartment and then out through the medicine outlet for the user to take the specified medicine; during that the corresponding medicine cartridge is pushed into the transition compartment, automatically determining remaining medicine quantity in the corresponding medicine cartridge by the medicine identification module and comparing with the medicine information in the system; comparing, by the system, medication dosage of the specified medicine and the remaining medicine quantity to determine whether to remind the user to replenish medicine via the prompt module; in response to the medication dosage being greater than the remaining medicine quantity, reminding the user to replenish medicine via the prompt module; in response to the medication dosage being less than or equal to the remaining medicine quantity, issuing no reminder; prompting, by the system, the user about the medication dosage based on the medicine information via the prompt module;
Operation 4: after the specified medicine is taken, de-energizing the push-rod electric motor, causing the corresponding medicine cartridge to retract into the transition compartment under an action of the springs; identifying the medicine information of the medicine in the corresponding medicine cartridge again by the medicine identification module and comparing with the medicine information in the system; comparing, by the system, whether the medicine in the corresponding medicine cartridge before and after the medication use is consistent; in response to the medicine in the corresponding medicine cartridge before and after the medication use being inconsistent, issuing an alarm via the prompt module; in response to the medicine in the corresponding medicine cartridge before and after the medication use being consistent, issuing no prompt; comparing, by the system, the remaining quantity of the medicine before and after the medication use to determine whether the user has completely taken the medication; in response to the remaining quantity before the medication use minus the remaining quantity after the medication use being not equal to a correct dosage, reminding the user of a dosage error via the prompt module; in response to the remaining quantity before the medication use minus the remaining quantity after the medication use being equal to the correct dosage, issuing no prompt, and driving the corresponding medicine cartridge to automatically retract into the medicine storage compartment.

The beneficial effects of the present disclosure, achieved through the application of the aforementioned technical solutions, are as follows.

In the intelligent medicine box and its usage method provided by the present disclosure, the rotatable frame is driven to rotate via the rotary motor such that the set medicine cartridge rotates to the medicine outlet, the medicine cartridge at the medicine outlet is driven out through the medicine outlet via the push-rod electric motor, the types and quantity of medicine in the medicine cartridge at the medicine outlet are identified via the identification module, the user is reminded of the dosage based on the types of medicine identified in the cartridge by the identification module via the prompt module, and the user is reminded of whether to replenish medicine based on the quantity of medicine identified in the cartridge by the identification module via the prompt module. Therefore, this intelligent medicine box has a simple and reliable structure, manages medicine accurately, can remind users to take medicine, remind users of the dosage according to the type of medicine, and remind users whether to replenish the medicine cartridge according to the quantity of medicine, making it convenient for elderly users to use.

### BRIEF DESCRIPTION OF THE DRAWINGS

FIG. 1 is a perspective schematic view of an intelligent medicine box according to the present disclosure.
FIG. 2 is a cross-sectional view of an intelligent medicine box according to the present disclosure.
FIG. 3 is a perspective schematic view of a drive module of an intelligent medicine box according to the present disclosure.
FIG. 4 is a perspective schematic view of a rotatable frame according to the present disclosure.
FIG. 5 is a perspective schematic view of a medicine cartridge according to the present disclosure.
FIG. 6 is a cross-sectional view of a medicine cartridge according to the present disclosure.

### DETAILED DESCRIPTION

The following describes the implementation of the present disclosure through specific embodiments. Those skilled in the art can easily understand other advantages and effects of the present disclosure from the content disclosed in this specification.

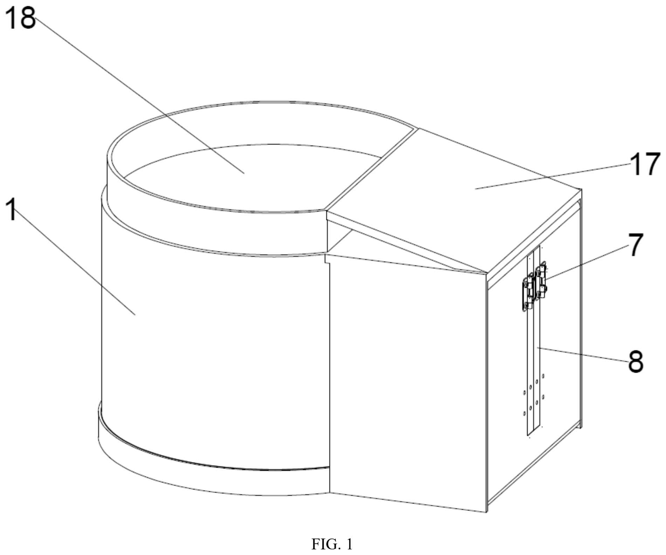

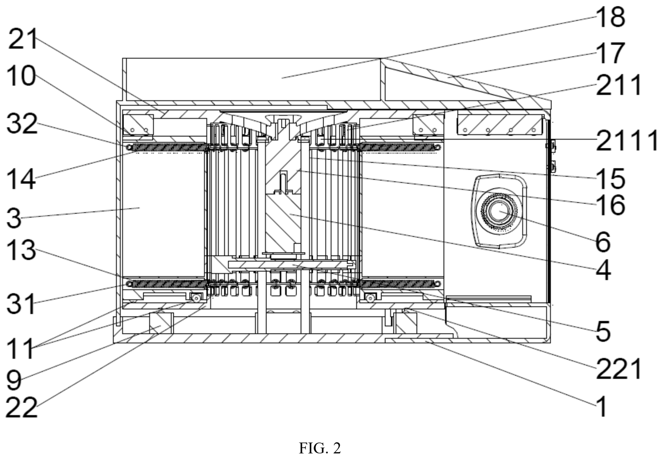

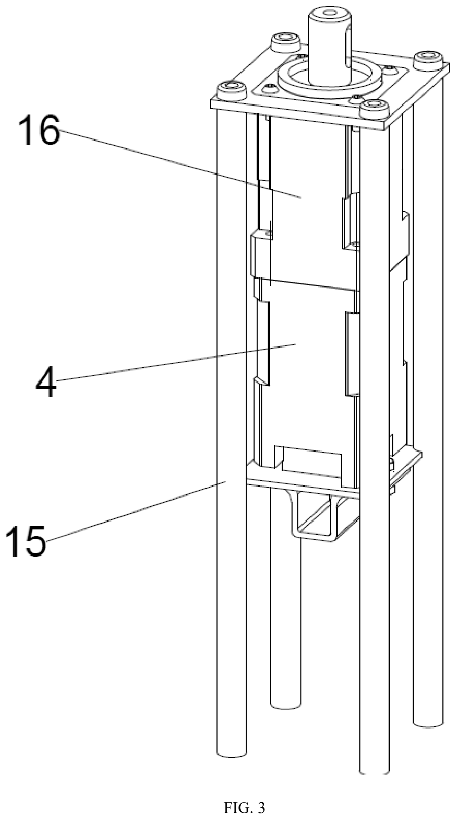

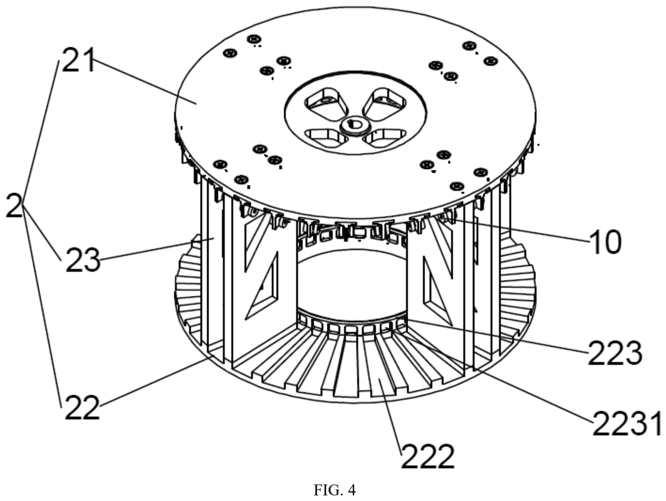

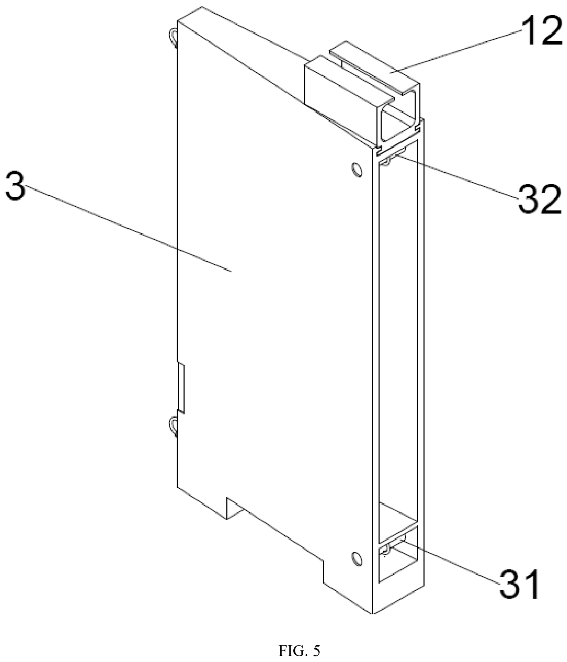

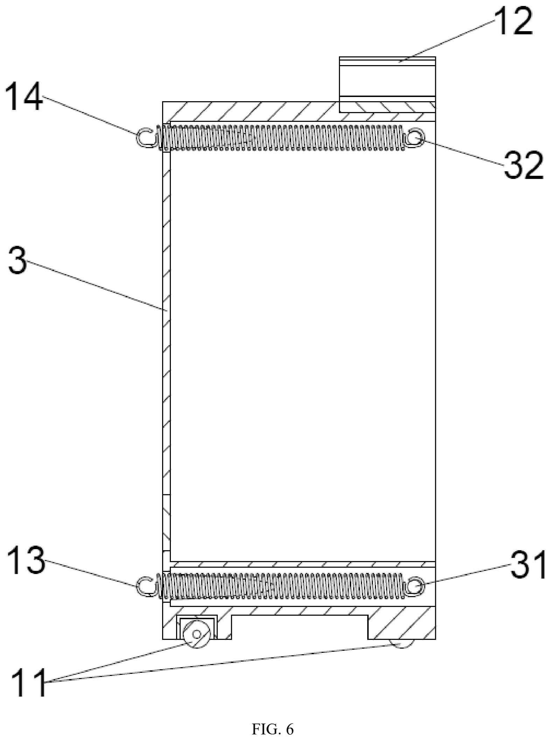

Referring to FIGS. 1-6, it should be noted that in the description of the present disclosure, terms such as "center", "upper", "lower", "left", "right", "vertical", "horizontal", "inner", "outer", etc., indicate orientation or positional relationships based on those shown in the accompanying drawings or the customary orientation or positional relationships when a product is in use. These terms are intended only to facilitate the description of the present disclosure and simplify the description, and do not indicate or imply that the referred device or component must have a specific orientation or be constructed and operated in a specific orientation. Therefore, they should not be construed as limiting the present disclosure. Furthermore, terms such as "first", "second", "third", etc., are intended only for distinguishing description and should not be understood as indicating or implying relative importance. Terms such as "horizontal", "vertical", "overhang", etc., do not require the components to be absolutely horizontal or overhanging but allow for slight inclination. For example, "horizontal" merely means that its direction is more horizontal relative to "vertical", and does not mean that the structure must be completely level; it may be slightly inclined.

In the description of the present disclosure, it should further be noted that, unless expressly specified and defined otherwise, terms such as "arranged", "mounted", "connected", and "linked" should be interpreted broadly. For example, it may be a fixed connection, a detachable connection, or an integral connection; it may be a mechanical connection, an electrical connection; it may be a direct connection, or an indirect connection through an intermediary medium; or it may be internal communication between two components. Those skilled in the art can understand the specific meanings of these terms in the present disclosure based on the specific context.

As shown in FIGS. 1 to 6, according to a general technical concept of the present disclosure, an intelligent medicine box is provided, including:
a medicine box body 1; where the medicine box body 1 is arranged with a medicine storage compartment and a transition compartment that are in communication with each other, and the medicine box body 1 is arranged with a medicine outlet in communication with the transition compartment;
a rotatable frame 2, rotatably arranged in the medicine storage compartment; where the rotatable frame 2 is arranged with multiple medicine cartridges 3 that are distributed peripherally and capable of reciprocating sliding radially; the multiple medicine cartridges 3 and the rotatable frame 2 are connected by springs that are radially arranged;
a drive module, fixedly arranged in the medicine box body 1 and including a rotary motor 4 and a push-rod electric motor 5; where the rotary motor 4 is configured to drive the rotatable frame 2 to rotate, causing one of the multiple medicine cartridge 3 to rotate to the medicine outlet; the push-rod electric motor 5 is configured to drive a push rod to push the one of the multiple medicine cartridge 3 that has rotated to the medicine outlet out through the medicine outlet;
a medicine identification module 6, fixedly arranged in the transition compartment and configured to identify medicine information;
a facial recognition module, fixedly arranged on the medicine box body 1 and configured to identify identity information of a user;
a prompt module, fixedly arranged on the medicine box body 1 and configured to remind the user to take medicine or replenish medicine; and
a control module 17, fixedly arranged on the medicine box body 1 and connected to the drive module, the medicine identification module 6, the facial recognition module, and the prompt module.

As shown in FIGS. 1 to 6, in some embodiments of the present disclosure, the medicine box body 1 is arranged with a medicine outlet door 8 at the medicine outlet, and the medicine outlet door 8 is installed via a torsion spring hinge 7. Under a normal condition, the medicine outlet door 8 is in a closed and sealed state, ensuring that an identification area is not affected by external light and preventing contamination and moisture inside the medicine box body 1. During a medicine dispensing process, the push-rod electric motor 5 pushes the designated medicine cartridge 3 out of the medicine outlet, thereby pushing open the medicine outlet door 8. When the push-rod electric motor 5 retracts and the medicine cartridge 3 re-enters the medicine box body 1, the medicine outlet door 8 automatically closes again.

As shown in FIGS. 1 to 6, in some embodiments of the present disclosure, the rotatable frame 2 includes a top plate 21, a bottom plate 22, and support plates 23. The top plate 21 is configured as a circular plate structure, the bottom plate 22 is configured as an annular plate structure, and the top plate 21 and the bottom plate 22 are arranged spaced apart vertically and facing each other and are fixedly supported by the support plates 23; a bottom of the medicine box body 1 is arranged with multiple ball seats 9 distributed at equal angles peripherally; a bottom side of the bottom plate 22 is arranged with an annular track 221 correspondingly matching balls on the ball seats 9, and the annular track 221 and the ball seats 9 constitute a guiding mechanism for guiding rotation of the rotatable frame 2.

As shown in FIGS. 1 to 6, in some embodiments of the present disclosure, a top side of the bottom plate 22 is arranged with multiple tracks 222 distributed at equal angles peripherally; a bottom side of the top plate 21 is arranged with multiple hanger wheels 10 distributed at equal angles peripherally; the multiple tracks 222 and the multiple hanger wheels 10 are arranged in a one-to-one correspondence; a bottom side of each medicine cartridge 3 is arranged with a pulley 11 to match a corresponding track 222, and a top side of the medicine cartridge 3 is arranged with a hanger rail 12 to match a corresponding hanger wheel 10.

As shown in FIGS. 1 to 6, in some embodiments of the present disclosure, an inner ring of the bottom plate 22 is arranged with a first spring mounting base 223 that is cylindrical; the first spring mounting base 223 defines multiple first spring connection hook holes 2231 matching the multiple tracks 222 in a one-to-one correspondence; the bottom side of the top plate 21 is arranged with a second spring mounting base 211 that is cylindrical; the second spring mounting base 211 defines multiple second spring connection hook holes 2111 matching the multiple hanger wheels 10 in a one-to-one correspondence; a first spring connection post 31 is arranged in and on a bottom of each medicine cartridge 3, and the first spring connection post 31 matches a corresponding first spring connection hook hole 2231; a first spring 13 is connected between the corresponding first spring connection hook hole 2231 and the first spring connection post 31; a second spring connection post 32 is arranged in and on a top of each medicine cartridge 3, and the second spring connection post 32 matches a corresponding second spring connection hook hole 2111; a second spring 14 is connected between the corresponding second spring connection hook hole 2111 and the second spring connection post 32. The push-rod electric motor 5 is configured to accomplish the feeding movement of the medicine cartridge 3, where the return of the medicine cartridge 3 is speed-controlled by the pulling force of the first spring 13 and the second spring 14 and the damping of the push-rod electric motor 5, and the feeding process is guided and constrained by the medicine cartridge 3's corresponding track 222 and hanger rail 12.

As shown in FIGS. 1 to 6, in some embodiments of the present disclosure, the drive module is fixedly arranged in the medicine box body 1 via a bracket 15 and disposed within the inner ring of the bottom plate 22, and the rotary motor 4 is connected to the rotation center of the top plate 21 in a transmission manner via a reduction gear 16.

As shown in FIGS. 1 to 6, in some embodiments of the present disclosure, the push-rod electric motor 5 is fixedly arranged in the medicine box body 1 via the bracket 15 and oriented toward the medicine outlet.

As shown in FIGS. 1 to 6, in some embodiments of the present disclosure, medicine in the medicine cartridge 3 is sheet-like or capsule-like medicine encapsulated in an aluminum-plastic blister pack. The medicine encapsulated in the aluminum-plastic blister pack is stored in the medicine cartridge 3 with a bottom of the medicine supported on the bottom of the medicine cartridge 3 and a top of the medicine resting against a side wall of the medicine cartridge 3; two medicine identification modules 6 are provided, which are respectively located on both sides of the medicine cartridge 3, thereby ensuring that the medicine can be identified by the medicine identification modules 6.

As shown in FIGS. 1 to 6, in some embodiments of the present disclosure, the medicine cartridge 3 is made of transparent plastic to allow the medicine identification module 6 to identify the information of the medicine in the cartridge. A top side of the medicine box body 1 further defines a medicine storage groove 18 for storing medicine bottles.

It should be noted that the control module 17 may further have a built-in communication unit, which can synchronize reminder information to a mobile terminal of a guardian, thereby facilitating the guardian to monitor medication use.

A usage method based on the aforementioned intelligent medicine box includes the following operations.

Operation 1: inputting the identity information of the user into a system through the facial recognition module; pushing one of the multiple medicine cartridges 3 out of the medicine box body 1 by the drive module, and placing medicine into the one of the multiple medicine cartridges 3; retracting the one of the multiple medicine cartridges 3 into the medicine box body 1 by the drive module; during a retraction process, automatically identifying information of the medicine in the one of the multiple medicine cartridges 3 by the medicine identification module 6, and inputting the information into the system; inputting a medication plan into the system through the control module 17.

Operation 2: according to the medication plan, reminding, by the system, the user to take medicine through the prompt module; in response to the user approaching the medicine box body 1, automatically identifying the identity information of the user by the facial recognition module; in response to the user being identified to be authorized, granting usage permission for the control module 17 to the user; in response to the user being identified to be unauthorized, denying the usage permission for the control module 17 to the user.

Operation 3: in response to medication use being authorized by the user through the control module 17, controlling, by the system, the rotary motor 4 to drive the rotatable frame 2 to rotate a corresponding medicine cartridge 3 corresponding to a specified medicine to a medicine dispensing position based on the information of the medicine; controlling, by the system, the push-rod electric motor 5 to drive the push rod to push the corresponding medicine cartridge 3 from the medicine dispensing position into the transition compartment and then out through the medicine outlet for the user to take the specified medicine; during that the corresponding medicine cartridge 3 is pushed into the transition compartment, automatically determining remaining medicine quantity in the corresponding medicine cartridge 3 by the medicine identification module 6 and comparing with the information of the medicine in the system; comparing, by the system, medication dosage of the specified medicine and the remaining medicine quantity to determine whether to remind the user to replenish medicine via the prompt module; in response to the medication dosage being greater than the remaining medicine quantity, reminding the user to replenish medicine via the prompt module; in response to the medication dosage being less than or equal to the remaining medicine quantity, issuing no reminder; prompting, by the system, the user about the medication dosage based on the information of the medicine via the prompt module.

Operation 4: after the specified medicine is taken, de-energizing the push-rod electric motor 5, causing the corresponding medicine cartridge 3 to retract into the transition compartment under an action of the springs; identifying the information of the medicine in the corresponding medicine cartridge 3 again by the medicine identification module 6 and comparing with the information of the medicine in the system; comparing, by the system, whether the medicine in the corresponding medicine cartridge 3 before and after the medication use is consistent; in response to the medicine in the corresponding medicine cartridge 3 before and after the medication use being inconsistent, issuing an alarm via the prompt module; in response to the medicine in the corresponding medicine cartridge 3 before and after the medication use being consistent, issuing no prompt; comparing, by the system, the remaining quantity of the medicine before and after the medication use to determine whether the user has completely taken the medication; in response to the remaining quantity before the medication use minus the remaining quantity after the medication use being not equal to a correct dosage, reminding the user of a dosage error via the prompt module; in response to the remaining quantity before the medication use minus the remaining quantity after the medication use being equal to the correct dosage, issuing no prompt, and driving the corresponding medicine cartridge 3 to automatically retract into the medicine storage compartment.

Therefore, the present disclosure has the following advantages:
In the intelligent medicine box and its usage method provided by the present disclosure, the rotatable frame is driven to rotate via the rotary motor such that the set medicine cartridge rotates to the medicine outlet, the medicine cartridge at the medicine outlet is driven out through the medicine outlet via the push-rod electric motor, the types and quantity of medicine in the medicine cartridge at the medicine outlet are identified via the identification module, the user is reminded of the dosage based on the types of medicine identified in the cartridge by the identification module via the prompt module, and the user is reminded of whether to replenish medicine based on the quantity of medicine identified in the cartridge by the identification module via the prompt module. Therefore, this intelligent medicine box has a simple and reliable structure, manages medicine accurately, can remind users to take medicine, remind users of the dosage according to the type of medicine, and remind users whether to replenish the medicine cartridge according to the quantity of medicine, making it convenient for elderly users to use.

The above embodiments are only illustrative of the principles and efficacy of the present disclosure and are not intended to limit the present disclosure. Those skilled in the art may modify or change the above embodiments without departing from the spirit and scope of the present disclosure. Therefore, all equivalent modifications or changes made by those skilled in the art without departing from the spirit and technical ideas disclosed in the present disclosure should still be covered by the claims of the present disclosure.

## Claims

1. An intelligent medicine box, comprising:
a medicine box body; wherein the medicine box body is arranged with a medicine storage compartment and a transition compartment that are in communication with each other, and the medicine box body is further arranged with a medicine outlet in communication with the transition compartment;
a rotatable frame, rotatably arranged in the medicine storage compartment; wherein the rotatable frame is arranged with a plurality of medicine cartridges that are distributed peripherally and capable of reciprocating sliding radially; the plurality of medicine cartridges and the rotatable frame are connected by springs that are radially arranged;
a drive module, fixedly arranged in the medicine box body and comprising a rotary motor and a push-rod electric motor; wherein the rotary motor is configured to drive the rotatable frame to rotate, causing one of the plurality of medicine cartridges to rotate to the medicine outlet; the push-rod electric motor is configured to drive a push rod to push the one of the plurality of medicine cartridges that has rotated to the medicine outlet out through the medicine outlet;
a medicine identification module, fixedly arranged in the transition compartment and configured to identify medicine information;
a facial recognition module, fixedly arranged on the medicine box body and configured to identify identity information of a user;
a prompt module, fixedly arranged on the medicine box body and configured to remind the user to take medicine or replenish medicine; and
a control module, fixedly arranged on the medicine box body and connected to the drive module, the medicine identification module, the facial recognition module, and the prompt module.

2. The intelligent medicine box according to claim 1, wherein the medicine box body is arranged with a medicine outlet door at the medicine outlet, and the medicine outlet door is installed via a torsion spring hinge.

3. The intelligent medicine box according to claim 1, wherein the rotatable frame comprises a top plate, a bottom plate, and support plates; the top plate is configured as a circular plate structure, the bottom plate is configured as an annular plate structure, and the top plate and the bottom plate are arranged spaced apart vertically and facing each other and are fixedly supported by the support plates;
a bottom of the medicine box body is arranged with a plurality of ball seats distributed at equal angles peripherally; a bottom side of the bottom plate is arranged with an annular track correspondingly matching the plurality of ball seats.

4. The intelligent medicine box according to claim 3, wherein a top side of the bottom plate is arranged with a plurality of tracks distributed at equal angles peripherally; a bottom side of the top plate is arranged with a plurality of hanger wheels distributed at equal angles peripherally; the plurality of tracks and the plurality of hanger wheels are arranged in a one-to-one correspondence; a bottom side of each medicine cartridge is arranged with a pulley to match a corresponding track, and a top side of the medicine cartridge is arranged with a hanger rail to match a corresponding hanger wheel.

5. The intelligent medicine box according to claim 3, wherein an inner ring of the bottom plate is arranged with a first spring mounting base that is cylindrical; the first spring mounting base defines a plurality of first spring connection hook holes matching the plurality of tracks in a one-to-one correspondence;
the bottom side of the top plate is arranged with a second spring mounting base that is cylindrical; the second spring mounting base defines a plurality of second spring connection hook holes matching the plurality of hanger wheels in a one-to-one correspondence;
a first spring connection post is arranged in and on a bottom of each medicine cartridge, and the first spring connection post matches a corresponding first spring connection hook hole; a first spring is connected between the corresponding first spring connection hook hole and the first spring connection post;
a second spring connection post is arranged in and on a top of each medicine cartridge, and the second spring connection post matches a corresponding second spring connection hook hole; a second spring 14 is connected between the corresponding second spring connection hook hole and the second spring connection post.

6. The intelligent medicine box according to claim 3, wherein the drive module is fixedly arranged in the medicine box body via a bracket and disposed within an inner ring of the bottom plate, and the rotary motor is connected to a rotation center of the top plate in a transmission manner via a reduction gear.

7. The intelligent medicine box according to claim 6, wherein the push-rod electric motor is fixedly arranged in the medicine box body via the bracket and oriented toward the medicine outlet.

8. The intelligent medicine box according to claim 1, wherein medicine in one of the plurality of medicine cartridges is sheet-like or capsule-like medicine encapsulated in an aluminum-plastic blister pack; the medicine encapsulated in the aluminum-plastic blister pack is stored in the one of the plurality of medicine cartridges, with a bottom of the medicine supported on a bottom of the one of the plurality of medicine cartridges and a top of the medicine resting against a side wall of the one of the plurality of medicine cartridges; two medicine identification modules are provided, which are respectively located on both sides of the one of the plurality of medicine cartridges.

9. The intelligent medicine box according to claim 1, wherein the medicine cartridge is made of transparent plastic, and a top side of the medicine box body further defines a medicine storage groove for storing medicine bottles.

10. A usage method based on the intelligent medicine box according to any one of claims 1-9, comprising:
Operation 1: inputting the identity information of the user into a system through the facial recognition module; pushing one of the multiple medicine cartridges out of the medicine box body by the drive module, and placing medicine into the one of the multiple medicine cartridges; retracting the one of the multiple medicine cartridges into the medicine box body by the drive module; during a retraction process, automatically identifying the medicine information of the medicine in the one of the multiple medicine cartridges by the medicine identification module, and inputting the medicine information into the system; inputting a medication plan into the system through the control module;
Operation 2: according to the medication plan, reminding, by the system, the user to take medicine through the prompt module; in response to the user approaching the medicine box body, automatically identifying the identity information of the user by the facial recognition module; in response to the user being identified to be authorized, granting usage permission for the control module to the user; in response to the user being identified to be unauthorized, denying the usage permission for the control module to the user;
Operation 3: in response to medication use being authorized by the user through the control module, controlling, by the system, the rotary motor to drive the rotatable frame to rotate a corresponding medicine cartridge corresponding to a specified medicine to a medicine dispensing position based on the medicine information; controlling, by the system, the push-rod electric motor to drive the push rod to push the corresponding medicine cartridge from the medicine dispensing position into the transition compartment and then out through the medicine outlet for the user to take the specified medicine; during that the corresponding medicine cartridge is pushed into the transition compartment, automatically determining remaining medicine quantity in the corresponding medicine cartridge by the medicine identification module and comparing with the medicine information in the system; comparing, by the system, medication dosage of the specified medicine and the remaining medicine quantity to determine whether to remind the user to replenish medicine via the prompt module; in response to the medication dosage being greater than the remaining medicine quantity, reminding the user to replenish medicine via the prompt module; in response to the medication dosage being less than or equal to the remaining medicine quantity, issuing no reminder; prompting, by the system, the user about the medication dosage based on the medicine information via the prompt module;
Operation 4: after the specified medicine is taken, de-energizing the push-rod electric motor, causing the corresponding medicine cartridge to retract into the transition compartment under an action of the springs; identifying the medicine information of the medicine in the corresponding medicine cartridge again by the medicine identification module and comparing with the medicine information in the system; comparing, by the system, whether the medicine in the corresponding medicine cartridge before and after the medication use is consistent; in response to the medicine in the corresponding medicine cartridge before and after the medication use being inconsistent, issuing an alarm via the prompt module; in response to the medicine in the corresponding medicine cartridge before and after the medication use being consistent, issuing no prompt; comparing, by the system, the remaining quantity of the medicine before and after the medication use to determine whether the user has completely taken the medication; in response to the remaining quantity before the medication use minus the remaining quantity after the medication use being not equal to a correct dosage, reminding the user of a dosage error via the prompt module; in response to the remaining quantity before the medication use minus the remaining quantity after the medication use being equal to the correct dosage, issuing no prompt, and driving the corresponding medicine cartridge to automatically retract into the medicine storage compartment.
